# EUROPÄISCHE PATENTSCHRIFT

(11) **EP 1 355 607 B1**
(45) Veröffentlichungstag und Bekanntmachung des Hinweises auf die Patenterteilung: **16.01.2008**
(21) Anmeldenummer: 01967183.3
(22) Anmeldetag: 16.07.2001
(51) Int. Cl.: A61F 13/472

(54) **INTERLABIALER HYGIENEARTIKEL**
INTERLABIAL HYGIENE ARTICLE
ARTICLE D'HYGIENE INTERLABIAL

(30) Priorität: 29.12.2000 DE 10065680
(43) Veröffentlichungstag der Anmeldung: 29.10.2003
(73) Patentinhaber: Hakle-Kimberly Deutschland GmbH, 55120 Mainz (DE)
(72) Erfinder: RAIDEL, Maria, 90451 Nürnberg (DE); ASCHENBRENNER, Franz, 92280 Kastl (DE)
(74) Vertreter: Zimmermann & Partner
(86) Internationale Anmeldenummer: PCT/EP2001/008194
(87) Internationale Veröffentlichungsnummer: WO 2002/053077

(56) Entgegenhaltungen:
- DE-U- 29 712 352
- FR-A- 2 420 339
- FR-A- 2 727 010
- GB-A- 2 296 437
- GB-A- 2 326 598
- US-A- 3 343 543
- US-A- 3 411 504
- US-A- 5 300 055
- US-A- 5 591 150
- US-A- 5 846 230

## Beschreibung

Die vorliegende Erfindung betrifft einen interlabialen Hygieneartikel.

FR-A-2727010 beschreibt eine Hygienebinde, die eine mittig in Längsrichtung verlaufende Erhebung und zwei im Mittelbereich in Längsrichtung verlaufende und in Querrichtung voneinander beabstandete Vertiefungen aufweist. Die Vertiefungen sind geeignet, die Labiae Maiorae aufzunehmen (Fig. 9).

FR-A-2420339 beschreibt eine Hygienebinde mit einer zentralen Erhöhuing, welche zwischen den Labiae Maiorae aufgenommen wird, und gegebenenfalls einem erhöhten Wulst entlang des Außenrands der Hygienebinde, der ein seitliches Austreten von Körperfluiden verhindern soll.

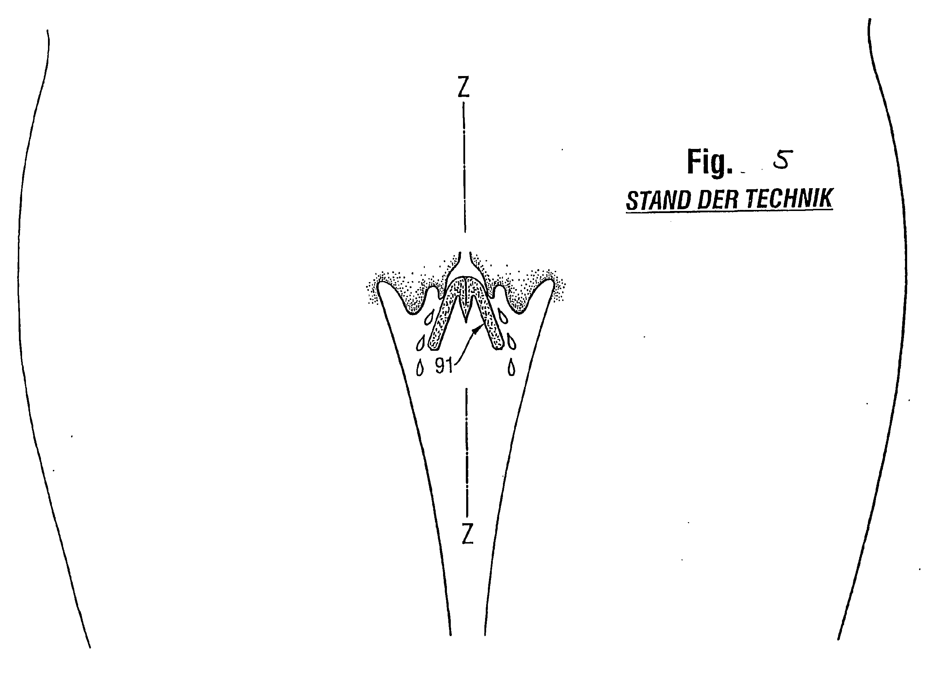

Ein bekannter interlabialer Hygieneartikel, der in Gebrauchsstellung im Querschnitt in Figur 5 gezeigt ist, weist den Nachteil auf, dass Körperflüssigkeit zwischen dem Hygieneartikel und den Labiae Externae sowie den Labiae Internae heruntersickern und -tropfen kann.

Der vorliegenden Erfindung liegt daher die Aufgabe zugrunde, einen interlabialen Hygieneartikel zu schaffen, der eine zusätzliche Sicherheit bietet und verhindern soll, dass Körperflüssigkeit (Menses, Urin und anderer Ausfluss) unerwünschterweise an die Beine, an die Unterwäsche, Bettzeug oder sonst nach aussen tritt.

Diese Aufgabe wird erfindungsgemäß durch den Gegenstand von Patentanspruch 1, gelöst. Bevorzugte Weiterbildungen sind in den abhängigen Ansprüchen, der Beschreibung und den Zeichnungen dargestellt.

Gemäß der vorliegenden Erfindung enthält der Absorptionskörper zwei längliche flache schalenförmige Elemente, deren Längsachsen in X-Richtung und deren Querachsen in Y-Richtung ausgerichtet sind, wobei die Unterseite des größeren schalenartigen Elements die Unterseite des Absorptionskörpers zumindest teilweise bildet. Unter der Bezeichnung flach ist in diesem Zusammenhang zu verstehen, dass die Höhe der schalenförmigen Elemente (in Z-Richtung gemessen) kleiner ist als deren Länge (in X-Richtung gemessen) und deren Breite (in Y-Richtung gemessen).

Der Rand des größeren schalenförmigen Elements bildet den Rand des Hygieneartikels. Das kleinere schalenförmige Element ist im Inneren des größeren schalenförmigen Elements derart angeordnet, dass deren Längsachsen im Wesentlichen miteinander fluchten.

Die Unterseite des kleineren schalenförmigen Elements ist an der Oberseite des größeren schalenförmigen Elements derart befestigt, dass zwischen der Außenwandung des kleineren schalenförmigen Elements und der Innenwandung des größeren schalenförmigen Elements eine ringförmige Ausnehmung entsteht. In die zumindest im Mittelbereich des Absorptionskörpers belegenen Abschnitte der ringförmigen Ausdehnung, die im Wesentlichen parallel zur Längsachse X verlaufen, sind die Labiae Maiorae einbringbar.

Gemäß eines weiteren Aspekts ist die Querdimension des inneren schalenförmigen Elements derart gewählt, dass der Rand des inneren schalenförmigen Elements zwischen Labia Maiora und Labia Minora anordbar ist.

Es ist zweckmäßig, wenn die Länge des kleineren schalenförmigen Elements 30% bis 70%, vorzugsweise 40% bis 60% von der Länge des größeren schalenförmigen Elements beträgt. Noch bevorzugter Weise beträgt deren Länge etwa die Hälfte der Länge des größeren schalenförmigen Elements, in X-Richtung gemessen.

Bevorzugterweise beträgt die Breite des kleineren schalenförmigen Elements 30% bis 70%, vorzugsweise 40% bis 60%, noch bevorzugter etwa die Hälfte der Breite des größeren schalenförmigen Elements, in Y-Richtung gemessen.

Gemäß eines speziellen Aspekts ist das kleinere, innen befindliche schalenförmige Element in Z-Richtung zumindest gleich hoch wie das größere schalenförmige Element.

Alternativ hierzu kann das kleinere schalenförmige Element höher sein als das größere schalenförmige Element, vorzugsweise 10% bis 50%.

Zweckmäßigerweise bestehen die Wandung des inneren schalenförmigen Elements und die Wandung des größeren schalenförmigen Elements aus absorbierendem Material.

Es ist besonders günstig, wenn zumindest die Unterseite des größeren schalenförmigen Elements mit einer flüssigkeitsundurchlässigen Schicht versehen ist.

Alternativ sind jedoch die Unterseiten beider schalenförmiger Elemente mit einer flüssigkeitsundurchlässigen Schicht versehen.

Eine weitere Alternative besteht darin, zumindest das obere schalenförmige ' Element aus hydrophoben Mehrschichtmaterialien, wie SMS, zu bilden oder deren Unterseite teildurchlässig zu gestalten bzw. diese zu hydrophobieren.

Vorteilhafterweise sind das kleinere und/oder das größere schalenförmige Element mit einer flüssigkeitsdurchlässigen Oberschicht und einem absorbierenden Kern, auch Saugkern genannt, und einer flüssigkeitsundurchlässigen Unterschicht versehen, die vorzugsweise im Gesamtbereich des Absorptionskörpers die gleiche Dicke haben. Es ist jedoch auch möglich, im Mittelbereich den absorbierenden Kern dicker oder mehrschichtig zu gestalten.

Bevorzugterweise ist die Unterschicht zweilagig ausgebildet, wobei eine flüssigkeitsundurchlässige Bahn auf ihrer distalen Seite mit einer weiteren körperfreundlichen Bahn versehen ist, die dem Hygieneartikel ein stoffartiges Aussehen und einen angenehmen Griff verleiht. Die körperfreundliche Bahn ist vorzugsweise eine Vliesstoffbahn.

Gemäß eines alternativen Aspekts ist die Wandung des kleineren schalenförmigen Elements, d. h. das absorbierende Material desselben, zumindest im Bodenbereich dicker als der Rest von dieser Wandung, um dort eine verstärkte Flüssigkeitsaufnahme zu erreichen.

Vorzugsweise besteht der Absorptionskörper aus einer Bahn eines blatt- oder bogenförmigen Materials, das zumindest auf seiner proximalen Seite absorbierend wirkt. Dieses wird im Rahmen dieser Anmeldung vereinfacht als absorbierendes Material bezeichnet. Zweckmäßigerweise ist der Absorptionskörper aus diesem Material gefaltet, geprägt, tiefgezogen, oder durch Wärme und Druck verformt.

Das Bahnmaterial enthält bevorzugt eine Schicht eines Absorbermaterials und fakultativ unter diesem eine flüssigkeitsundurchlässige untere Schicht und/oder auf diesem eine flüssigkeitsdurchlässige obere Deckschicht.

Vorzugsweise ist das Bahnmaterial biegsam und gleichzeitig eigensteif.

Gemäß einer weiteren Variante ist die Oberseite des Absorptionskörpers mit einer an sich bekannten, in Längsrichtung verlaufenden Plissierung versehen.

Als zweckmäßig hat es sich ferner erwiesen, wenn der äußere Rand des Absorptionskörpers nach innen oder nach außen umgebogen ist.

Es ist günstig, wenn dabei der äußere Rand des Absorptionskörpers nach außen umgebogen ist.

Nach einem zusätzlichen Aspekt besteht der Absorptionskörper aus einer Schicht eines absorbierenden Materials, die zwischen einer flüssigkeitsdurchlässigen Schicht und einer flüssigkeitsundurchlässigen Schicht angeordnet ist, wobei ein Randbereich die flüssigkeitsundurchlässige Schicht um das freie Ende der Schicht aus absorbierendem Material und der Schicht aus flüssigkeitsdurchlässigem Material herumgelegt und an letzteres befestigt ist.

Die in den verschiedenen Aspekten beschriebene Schicht aus flüssigkeitsdurchlässigem Material dient als Deckschicht. Sie ist hinlänglich porös, um flüssigkeitsdurchlässig zu sein, wobei sie Flüssigkeit rasch durch ihre Dicke durchtreten läßt. Eine geeignete Schicht kann aus einer Vielzahl von Bahnmaterialien, beispielsweise aus porösen Schaumstoffen, retikulierten Schaumstoffen, Lochkunststoffolien, Naturfasern (beispielsweise Holz oder Baumwollfasern), aus Kunstfasern (beispielsweise Polyester- oder Polypropylenfasern) oder einer Kombination aus Natur- und Kunstfasern hergestellt werden. Diese Schicht wird für gewöhnlich auch verwendet, um ein Rücknässen zu minimieren und ein angenehm weiches komfortables Tragegefühl zu gewährleisten.

Für die Deckschicht können verschiedene Gewebe- und Vliesstoffe verwendet werden. Beispielsweise kann die Deckschicht aus einer schmelzgeblasenen oder spinngebundenen Bahn aus Polyolefinfasern zusammengesetzt sein. Die Deckschicht kann auch eine gebundene kardierte Bahn aus Natur- und.Kunstfasern sein.

Auch Vliesstoffbahnen kommen hierfür in Frage. Eine "Vliesstoffbahn" ist eine Bahn aus Material, die ohne Zuhilfenahme eines textilen Web- oder Wirkverfahrens gebildet wird.

Die Deckschicht kann aus einem im Wesentlichen hydrophoben Material zusammengesetzt sein, und das hydrophobe Material kann wahlweise mit einem oberflächenaktiven Stoff behandelt oder auf andere Weise bearbeitet werden, um ihm einen gewünschten Grad an Benetzbarkeit und Hydrophilität zu verleihen. Die Deckschicht kann auch ein spinngebundener Polypropylen-Vliesstoff sein, der sich aus Fasern mit ungefähr 2,8 - 3,2 Denier zusammensetzt, die zu einer Bahn geformt sind, welche ein Flächengewicht von ungefähr 22 g/m² und eine Dichte von ungefähr 0,06 g/cm³ aufweist. Der Stoff kann mit flächenaktiven Stoffen wie etwa 0,28% des oberflächenaktiven Stoffs Triton X-102 behandelt sein.

Die Deckschicht kann ferner aus einer gewellten oder plissierten Bahn bestehen, wie sie Z. B. in der Patentanmeldung WO96/00625 beschrieben sind.

Besonders bevorzugte Materialien zur Herstellung dieser Vliessstoffe sind auf Seite 17, Zeile 30 bis 34 der vorgenannten WO96/00625 beschrieben. Die gewellten Bahnen sind bevorzugt derart auf der Oberseite des Absorptionskörpers angebracht, dass die einzelnen Falten in Längsrichtung X verlaufen und Förderkanäle für die Flüssigkeit bilden. Alternativ ist es jedoch möglich, die Bahnen so auf der Oberseite des Absorptionskörpers anzubringen, dass sie in Querrichtung Y orientiert sind, was einer Ausdehnung aufgenommener Flüssigkeit in Längsrichtung des Absorptionskörpers entgegenwirkt.

Die rückseitige Schicht kann zwar aus einem flüssigkeitsdurchlässigen Material gebildet sein, umfaßt jedoch vorzugsweise ein Material, welches im Wesentlichen flüssigkeitsundurchlässig ist. Beispielsweise kann eine typische rückseitige Schicht aus einer dünnen Kunststoffolie oder aus einem anderen flexiblen flüssigkeitsundurchlässigen Material hergestellt werden. Der Begriff "flexibel" bezeichnet Materialien, die anschmiegsam sind und sich problemlos der allgemeinen Form und den allgemeinen Konturen des Körpers des Trägers anpassen. Die rückseitige Schicht verhindert, daß die in der saugfähigen Struktur eingeschlossenen Exudate Artikel, wie Unterwäsche, Bettwäsche und Oberbekleidung, die in Kontakt mit dem Hygieneprodukt treten, benetzen. Die rückseitige Schicht kann eine einheitliche Materialschicht sein oder eine aus mehreren Seite an Seite zusammengesetzten oder laminierten Komponenten bestehende Verbundschicht.

Bei einer Ausgestaltung ist die rückseitige Schicht eine Polyethylenfolie mit einer Dicke von ungefähr 0,012 Millimeter bis ungefähr 0,051 Millimeter, speziell eine Folie mit einer Dicke von ungefähr 0,032 Millimeter. Alternative Konstruktionen der rückseitigen Schicht können eine Gewebe- oder Vliesstoffbahn umfassen, die zur Gänze oder zum Teil behandelt wurde, um ausgewählten Bereichen, die dem saugfähigen Körper benachbart oder in dessen Nähe liegen, den gewünschten Grad an Flüssigkeitsundurchlässigkeit zu verleihen.

Es ist besonders günstig, wenn die rückseitige Schicht aus einem biologisch abbaubaren oder in Wasser sich allmählich zersetzendem Material besteht, um eine bequeme Entsorgung über die Toilette zu ermöglichen. Gleiches gilt selbstverständlich auch für die obere Schicht und die Absorberschicht.

Die rückseitige Schicht bildet für gewöhnlich die äußere Abdeckung des Hygieneartikels. Wahlweise kann die rückseitige Schicht eine oder mehrere getrennte Schichten umfassen, welche zusätzlich zur äußeren Abdeckungsschicht vorgesehen werden und zwischen der äußeren Abdeckungsschicht und der saugfähigen Struktur angeordnet werden können.

Die rückseitige Schicht kann wahlweise aus einem mikroporösen, "atmungsaktiven" Material gebildet sein, welches Wasserdampf entweichen läßt, der verhindert, daß Körperflüssigkeiten durch die rückseitige Schicht treten. Beispielsweise kann die atmungsaktive rückseitige Schicht aus einer mikroporösen Polymerfolie oder einem Vliesstoff bestehen, welche beschichtet oder auf andere Weise behandelt wurden, um diesen den gewünschten Grad an Flüssigkeitsundurchlässigkeit zu verleihen. Beispielsweise seien genannt eine mikroporöse Folie aus PMP-1-Material, von Mitsui Toatsu Chemicals, Inc., einer Gesellschaft mit Firmensitz in Tokio, Japan, oder eine Polyolefinfolie XKO-8044, die bei der in Minneapolis, Minnesota, ansässigen Firma 3M erhältlich ist. Die rückseitige Schicht kann auch geprägt oder auf andere Weise mit einer matten Oberfläche versehen werden, um ihr ein gefälligeres äußeres Erscheinungsbild zu verleihen.

Die absorbierende Schicht besteht vorzugsweise aus einer saugfähigen Struktur, auch Saugkern genannt, die ein absorbierendes d. h. saugfähiges Material enthält, das zwischen der Deckschicht und der rückseitigen Schicht angeordnet wird, um den Absorptionskörper zu bilden. Der Saugkern weist eine Konstruktion auf, die im allgemeinen zusammendrückbar und anschmiegsam ist und die Haut des Trägers nicht reizt. Die saugfähige Struktur ist vorzugsweise einteilig. Es kann aber auch alternativ aus der Vielzahl einzelner Materialien gebildet sein, welche aneinander befestigt sind.

Es ist auch möglich, die saugfähige Struktur ohne darüberliegende flüssigkeitsdurchlässige Deckschicht zu verwenden, wozu das saugfähige Material vorzugsweise an der Oberseite der Struktur verdichtet ist.

Verschiedene Arten von benetzbarem, hydrophilem Fasermaterial können verwendet werden, um die saugfähige Struktur zu bilden. Geeignete Fasern sind natürlich vorkommende organische Fasern, aus benetzbarem Material, beispielsweise Cellulosefasern; Kunstfasern, die aus Cellulose oder Cellulosederivaten erhalten sind, beispielsweise Rayonfasern; anorganische benetzbare Fasern wie Glasfasern; Kunstfasern, aus benetzbaren Thermoplast-Polymeren, beispielsweise spezielle Polyester- oder Polyamidfasern; und Kunstfasern, die aus einem nichtbenetzbaren Thermoplastpolymer zusammengesetzt sind, beispielsweise Polypropylenfasern, welche durch geeignete Mittel hydrophiliert sind. Die Fasern können durch Behandeln mit Silicamaterial, Behandeln mit einem Material, das eine geeignete hydrophile Komponente aufweist und nicht einfach von der Faser entfernt werden kann, oder durch Umhüllen der nichtbenetzbaren hydrophoben Faser mit einem hydrophilen Polymer während oder nach der Bildung der Faser hydrophiliert werden. Auch Mischungen der verschiedenen oben genannten Faserarten können zum Einsatz kommen:

Der Begriff "hydrophil" betrifft Fasern oder die Oberflächen von Fasern, welche durch die wäßrigen Flüssigkeiten, die mit den Fasern in Kontakt stehen, benetzt werden. Der Benetzungsgrad der Materialien kann wiederum mit Hinblick auf die Randwinkel und die Oberflächenspannungen der beteiligten Flüssigkeiten und Materialien beschrieben werden. Fasern, die Randwinkel von weniger als 90° aufweisen, werden als "benetzbar" bezeichnet, wohingegen Fasern, deren Randwinkel größer als 90° sind, als "nichtbenetzbar" bezeichnet werden.

Zumindest teilweise als Granulat vorliegende Cellulosefasern können eine geeignete absorbierende Struktur bilden, wenn diese Fasern zwischen einer flüssigkeitsdurchlässigen proximalen und einer flüssigkeitsundurchlässigen distalen unteren Schicht angebracht sind. Das Granulat erlaubt eine gute Anpassung an die individuelle Anatomie der Trägerin. Ein überlappendes seitliches Verrutschen des Cellulosematerials kann durch entsprechende Unterteilungen in abgeschlossene Bereiche verhindert werden.

Das Material aus zumindest teilweise als Granulat vorliegenden Cellulosefasern ist rieselfähig und bleibt auch nach Beaufschlagung mit einer Flüssigkeit rieselfähig. Dadurch wird außerdem erreicht, dass die Funktionalität des Artikels auch in einem deformierten Zustand aufrechterhalten wird.

Die zumindest teilweise als Granulat vorliegenden Cellulosefasern können aus Holz und/oder anderen Pflanzenfasern gewonnen werden. Dabei werden die aus Holz gewonnenen zumindest teilweise als Granulat vorliegenden Cellulosefasern bevorzugt. Andere, ebenfalls geeignete Pflanzenfasern sind zum Beispiel Apfelfasern, Orangenfasern und Weizenfasern. Die Herstellung von zumindest teilweise als Granulat vorliegenden Cellulosefasern aus Holz und/oder anderen Pflanzenfasern ist dem Fachmann bekannt.

Das Material bleibt bis zu einer Flüssigkeitsbeaufschlagung von mindestens 10 ml/g Material rieselfähig. Dies ermöglicht eine Anschmiegung an die jeweilige Körperform des Trägers während unterschiedliche Bewegungen und Belastungsarten möglich sind.

Das Granulat weist einen wollbällchenähnlichen Aufbau auf. Dadurch enthalten sie ein gewisses "void volume", also einen Hohlraum, der zu einer großen Oberfläche führt. Dieser spezielle Aufbau des Granulats trägt vermutlich zu der höheren Beweglichkeit und Rieselfähigkeit bei, sorgt aber auch für Hohlräume, in denen Flüssigkeiten, insbesondere z.B. Blutzellen, aufgefangen und gespeichert werden können.

Das Granulat kann in eine Matrix aus Fasermaterial eingelagert sein. Dabei kann das Material homogen in die Fasermatrix eingemischt werden, so dass die Bestandteile des Materials, welches auch nach Beaufschlagung mit einer Flüssigkeit rieselfähig bleibt, gleichmäßig über die Faserstruktur verteilt und in dieser eingelagert sind. Alternativ dazu kann der Saugkörper aber auch einen schichtartigen Aufbau zeigen. Materialien, welche als Faserstoffe für die vorgenannten Zwecke besonders geeignet sind, sind Zellstoff oder eine Mischung aus Zellstoff und Polypropylen, d. h. ein sogenanntes Coform-Material. Durch das Fasermaterial wird eine noch optimalere Flüssigkeitsverteilung in dem erfindungsgemäßen absorbierenden Artikel erreicht, da die Fasern eine bestimmte Saugfähigkeit aufweisen und Flüssigkeit gerichtet transportieren können. Das Verhältnis von saugfähigem Material, welches auch nach Beaufschlagung mit einer Flüssigkeit rieselfähig bleibt, und Fasermaterial beträgt vorzugsweise 1 bis 25 Gew.-% zu 99 bis 75 Gew.-% und insbesondere 10 bis 15 Gew.-% zu 90 bis 85 Gew.-%.

Die zumindest teilweise als Granulat vorliegenden Cellulosefasern können vollständig in Form eines Granulats, im wesentlichen in Form eines Granulats oder als ein Gemisch aus Fasern und Granulat vorliegen. Sowohl das Granulat als auch das Gemisch sind rieselfähig, verbunden mit den oben beschriebenen Vorteilen.

Gemäß einer bevorzugten Ausführungsform weisen die Partikel in dem Granulat einen Durchmesser von 50 bis 3000 µm auf.

Besonders bevorzugt weisen sie einen Durchmesser von 400 *µ*m auf.

Weiterhin bevorzugt ist ein mittleres Schüttgewicht für das Granulat (gemessen gemäß DIN 53 468) von 30 bis 600 g/l Besonders bevorzugt wird hier ein mittleres Schüttgewicht von 100-300 g/l +/- 15 %.

Beispielsweise handelt es sich bei dem Granulat um Granulat aus dem Produkt LC 200 - Cellulosefasern der Firma J. Rettenmaier + Söhne GmbH + Co., DE.

Die dem Körpergewebe zugewandte, d.h. proximale, Schicht des Absorptionskörpers ist vorzugsweise derart behandelt, dass ein Austrocknen der Schleimhäute verhindert wird. Beispielsweise ist hier eine Glycerinbeschichtung verwendet; auch andere Lotionen oder Gleitmittel, wie sie bei feuchtem Toilettenpapier üblich sind, können hier zum Einsatz kommen. Auch körpergeruchsüberdeckende oder neutralisierende Substanzen, wie Ringelblume, Kamille und Aloe, oder medizinische Substanzen, z.B. zur Entzündungshemmung oder ähnliches, können auf oder in dieser Schicht aufgebracht sein.

Die Länge des Absorptionskörpers beträgt bei den vorstehend beschriebenen Varianten und Abwandlungen von diesen in X-Richtung gemessen vorzugsweise 30 bis 150 mm, noch bevorzugter Weise 40 - 120 mm. Dabei beträgt die Länge des Mittelbereichs in X-Richtung gemesssen vorzugsweise 15 bis 90 mm, noch bevorzugter Weise 25 bis 60 mm. Die Höhe des Absorptionskörpers gemäß den vorstehend beschriebenen Varianten und Abwandlungen von diesen beträgt in Z-Richtung gemessen vorzugsweise 4 bis 30 mm noch bevorzugter Weise 6 bis 20 mm. Die vorstehend erwähnten Maßangaben verstehen sich für Absorptionskörper, die vor Gebrauch frei auf einer ebenen Oberfläche liegen und wie man sie beispielsweise erhält, wenn man die Absorptionskörper aus einer Packung entnimmt in der eine Mehrzahl von ihnen enthalten ist und in der sie aus Platzersparnisgründen für den Transport zusammengedrückt waren.

Die vorzugsweise seitlich in der Packung zusammengedrückten Absorptionskörper versuchen nach ihrer Freisetzung ihre ursprüngliche Gestalt wieder anzunehmen, bedingt durch die Elastizität der verwendeten Materialien und die spezielle oben beschriebene Formgebung.

Nach der Freisetzung ist in kurzer Zeit der gemessene stabile Endzustand erreicht.

Bei denjenigen Ausführungsformen, bei welchen der Absorptionskörper ein weich nachgiebiges Kissen ist, beträgt die Breite des Absorptionskörpers (in Y-Richtung gemessen) bei der vorherbeschriebenen Meßmethode vorzugsweise 10 bis 60 mm, noch bevorzugter Weise 20 bis 50 mm. Bei den gefalteten Varianten verbleibt ohne manuelle Nachhilfe der Absorptionskörper nach seiner Entnahme aus der Packung unter Umständen zusammengeklappt und muß vor dem Einführen erst in Breitenrichtung angepaßt werden.

Es ist jedoch zu beachten, dass die verwendeten Materialien nicht starr sind, so dass die Breite und auch die Höhe von der Benutzerin ihren anatomischen Gegebenheiten entsprechend leicht angepasst werden kann. Die Nachgiebigkeit der Materialien und die spezielle Formgestaltung der Absorptionskörper kommen einem seitlichen Zusammendrücken und bei vielen Varianten auch einem Auseinanderziehen entgegen. Ein seiltiches Auseinanderziehen sollte bevorzugt bis zu einer Breite von ca. 60 mm möglich sein.

Die Dicke des absorbierenden Kissens beträgt vorzugsweise 2 bis 30 mm, noch bevorzugter Weise etwa 3 bis 20 mm. Bei Verwendung des in den anderen Varianten gezeigten Bahnmaterials beträgt die Dicke vorzugsweise 0,1 bis 10 mm, noch bevorzugter Weise etwa 1 bis 5 mm.

Die Breite der Ausnehmungen oder Gräben beträgt im Mittelbereich des Absorptionskörpers vorzugsweise 5 bis 30 mm, noch bevorzugter Weise 15 bis 20 mm.

Das Absorptionsvermögen des Absorptionskörpers beträgt vorzugsweise 0,5 g bis 15 g. Es wird mit einer Blutersatzflüssigkeit gemessen bei einer Belastung von 2 kg.

Im Folgenden ist das Prüfverfahren näher beschrieben.

Der Absorptionskörper wird zunächst insgesamt mit einer Waage gewogen, die eine Genauigkeit von +/- 10 mg aufweist.

Ein Sieb mit Außenabmessungen von 150 x 120 mm, Siebdicke 1,5 mm, Lochabstand 1,5 mm und einem Lochdurchmesser von 2 mm trägt einen Plexiglasrahmen (Höhe 20 mm, Flächeninnenmaß 100 x 100 mm), der auf das Sieb geschraubt ist und auf den Seiten mit 16 mm hohen Schlitzen versehen ist zum leichten Abfluss der Blutersatzflüssigkeit. Der Absorptionskörper wird innerhalb des Plexiglasrahmens auf das Sieb gelegt.

Die Rezeptur für die Blutersatzlösung ist folgende:

| Einwaage | n | g/l |
|---|---|---|
| 14,0 | g | Tylose H 20p der Fa. Hoechst |
| 100,0 | g | Glycerin |
| 10,0 | g | Kochsalz (NaCl) |
| 4,0 | g | NaHCO₃ |
| 2,0 | g | Duasyn-Säurepanceau 4RC von Cassella |
| 2,0 | g | Konservierungsmittel CA 24 |
| | | (Firma Biochema; Schwaben, Dr. Lehmann & Co.) |

Auf den Absorptionskörper wird anschließend eine Al-Platte gelegt mit einem Flächenmaß von 100 x 65 mm und einem Gewicht von ca. 45 g, um etwaige Luftblasen herauszudrücken.

Der auf dem Sieb ruhende Absorptionskörper wird sodann in die Blutersatzlösung eingetaucht und verbleibt in dieser 10 Minuten. Danach wird die Blutersatzlösung aus dem Behälter abgelassen bis die Oberfläche der Blutersatzlösung unter die Unterkante des Siebes abgesunken ist. Nach einer Wartezeit von 60 Sekunden wird die A1-Platte entfernt. Auf die nasse Probe wird sodann ein Plexiglasstempel mit einem Flächenmaß von 99 x 99 mm, Höhe 25 mm, Gewicht ca. 60 g gelegt, auf den anschließend ein Gewicht von 2 kg mit einem Flächenmaß von 110 x 110 mm gelegt wird.

Danach wird die Blutersatzlösung wieder aufgefüllt, so dass der Absorptionskörper voll in diese eintaucht. Nach 10 Minuten Wartezeit wird die Blutersatzlösung wieder abgezogen, bis die untere Siebkante erreicht ist. Nach einer Wartezeit von 60 Sekunden werden das Gewicht und der Plexiglasstempel abgenommen und die mit Hilfe einer Pinzette herausgehobene Probe wird abermals gewogen. Die Differenz zwischen dieser Wägung und der Wägung des trockenen Absorptionskörpers definiert das Absorptionsvermögen des Absorptionskörpers.

Die beiliegenden Figuren dienen der weiteren Erläuterung der Erfindung. Es zeigen:
- Fig. 1: eine perspektivische, in der Mitte quer durchgeschnittene Darstellung eines erfindungsgemäßen Hygieneartikels.
- Fig. 2: einen Längsschnitt längs der Linie VI - VI von Fig. 1 der dort gezeigten Variante des Hygieneartikels.
- Fig. 3A, 3B: vergrößerte Detaildarstellungen von zwei Varianten des Randes des gezeigten Hygieneartikels.
- Fig. 4: in schematisierter Darstellung die Benutzung des in Fig. 1 und Fig. 2 dargestellten interlabialen Hygieneartikels.
- Fig. 5: zeigt eine Darstellung entsprechend der von Fig. 4 von einem interlabialen Hygieneartikel nach dem Stand der Technik.

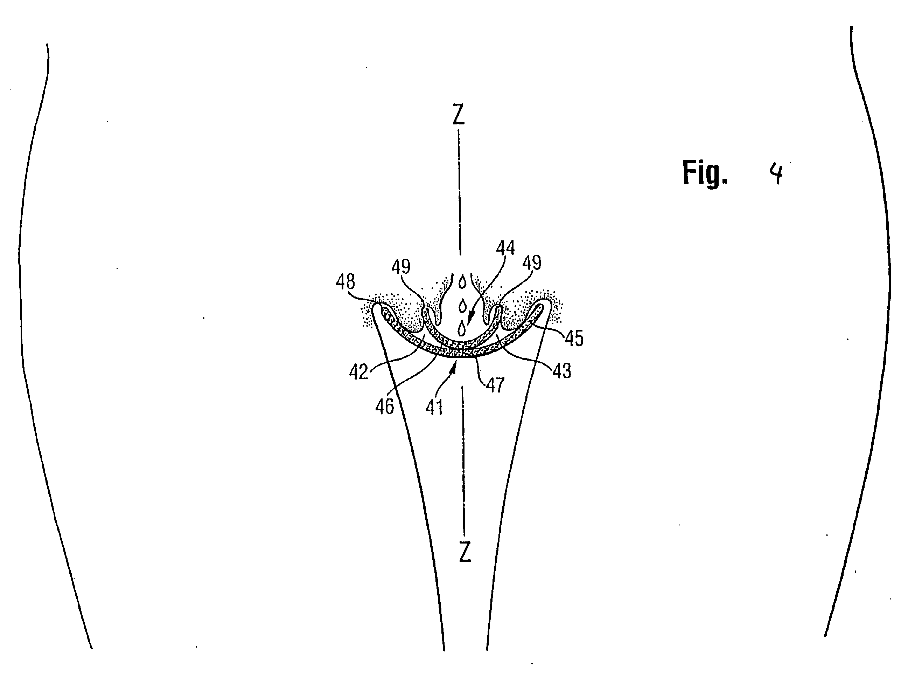

Der erfindungsgemäße Hygieneartikel soll vorab generell anhand der Fig. 4 kurz erläuteret werden. Die Figur zeigt im Schnitt den unteren Bereich eines weiblichen Torsos einschließlich der beiden Labiae Majorae, der beiden Labiae Minorae (auch Labiae Internae genannt) und des Vestibulums (Vestibül). Man erkennt, dass die Vertiefungen des Absorptionskörpers 41 d. h. die Vertiefungen 42, 43 die beiden Labiae Majorae aufnehmen. Der Bereich zwischen den Vertiefungen 42, 43 enthält eine zusätzliche mittlere Vertiefung 44 wobei die Begrenzung je zweier aneinander angrenzenden Vertiefungen jeweils zwischen Labia Majora und Labia Minora zu liegen kommen. Die mittlere Vertiefung fängt gefäßartig die aus dem Vestibulum heraustropfende oder entlanglaufende Körperflüssigkeit auf und absorbiert diese. Im Gegensatz zu dem in Fig. 5 gezeigten Hygieneartikel 91 nach dem Stand der Technik wird ein seitliches Herabfliessen der Körperflüssigkeit längs der Außenfläche des interlabialen Hygieneartikels 91 durch die erfindungsgemäßen Version gemäß Fig. 4 sicher vermieden.

Etwaige über den Rand der mittleren Vertiefung 44 hinaustretende Flüssigkeit wird in den äußeren Vertiefungen 42, 43, sicher aufgesaugt.

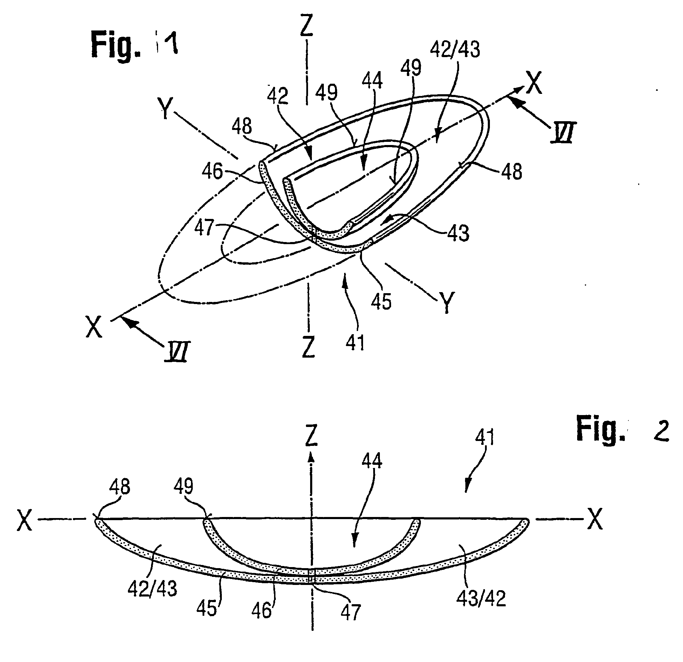

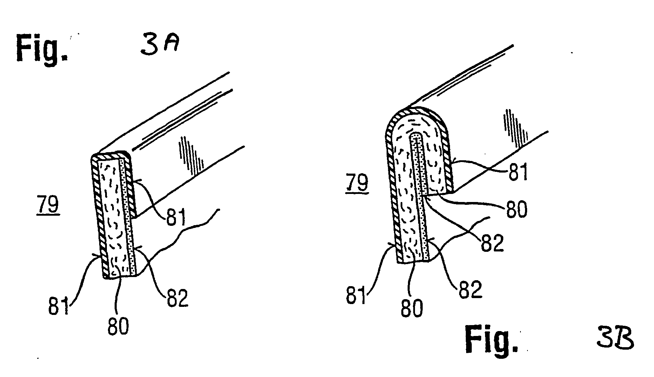

Der in Fig. 1 und 2 gezeigte Hygieneartikel enthält als Absorptionskörper 41 zwei längliche, flache schalenförmige Elemente 45 und 46, von denen das kleinere Element 46 mittig in dem größeren Element 45 angeordnet ist, derart; dass die Längsachsen miteinander und mit der X-Achse fluchten. Das innere kleinere schalenförmige Element 46 ist in seiner Mitte an der Unterseite mit der Oberseite des größeren schalenförmigen Elements 45 verbunden, wie dies durch das Bezugszeichen 47 angedeutet ist. Die oberen Ränder 48, 49 der beiden schalenförmigen Elemente 45 und 46 liegen bei diesem Ausführungsbeispiel auf gleicher Höhe. Alternativ ist vorgesehen, den Rand 49 des inneren schalenförmigen Elements 46 höher hinaufzuführen, als den Rand 48 des größeren und äußeren schalenförmigen Elements 45.

Die im Mittelbereich des Absorptionskörpers belegenen seitlichen Ausnehmungen 42 und 43 vereinigen sich in den Endbereichen und bilden somit eine umlaufende Ausnehmung 42/43, die zur Aufnahme der beiden Labiae Maiorae dient, während, wie in Fig. 4 gezeigt, der obere Rand 49 des kleineren schalenförmigen Elements 46 in den Bereich zwischen Labia Maiora und Labia Minora hinaufreicht und die mittlere Ausnehmung 44 Menstruations- und andere Körperflüssigkeiten aufnimmt.

Da beide schalenförmigen Elemente 45 und 46 aus einem absorbierenden blatt- oder bogenförmigen Material oder aus einem dünnen, kissenartigen, absorbierenden Material bestehen, wird die Körperflüssigkeit von den Elementen aufgesaugt. Wie auch bei den anderen Varianten können die schalenförmigen Elemente allein aus absorbierendem Material bestehen oder zusätzlich eine darunterliegende flüssigkeitsundurchlässige Schicht und/oder eine darüberliegende flüssigkeitsdurchlässige Schicht aufweisen, die jedoch aus Gründen der Übersichtlichkeit nicht dargestellt sind.

In den Fig. 3A und 3B sind zwei Varianten des Randbereichs 79 wiedergegeben, wobei in diesen Beispielen der Absorptionskörper aus einem dreischichtigen Material besteht, einem absorbierenden Kernmaterial 80, das auf seiner rückwärtigen oder distalen Seite mit einer Schicht 81 eines flüssigkeitsundurchlässigen Materials überzogen oder kaschiert ist und auf seiner Oberseite oder proximalen Seite eine flüssigkeitsdurchlässige Schicht 82 aufweist.

In der Variante von Fig. 3A ist das Ende der flüssigkeitsundurchlässigen Schicht 81 über das Ende der absorbierenden Schicht 80 und der flüssigkeitsdurchlässigen Schicht 82 herumgebogen. Dies kann nach innen, wie in Fig. 3A gezeigt, aber auch nach aussen erfolgen.

In der Variante von Fig. 3B sind die freien Enden der Schichten 80, 81, 82 gemeinsam umgebogen.

Die sogenannte "Anatomisierung" der Absorptionskörper im Bereich der weiblichen Anatomie soll nur allgemeine und schematische Darstellungen andeuten. In Realitas formt sich das Absorptionsmaterial während des Gebrauchs selbstverständlich innig und umfassend in die Körperoberflächen, -vertiefungen und -höhlen ein insbesondere nachdem es mit entsprechender Körperflüssigkeit beaufschlagt ist bzw. die Absorption begonnen hat.
- X: X-Achse, längs
- Y: Y-Achse, quer
- Z: Z-Achse, hoch

- 41: Absorptionskörper (Fig. 1, 2 und 4)
- 42: seitliche Ausnehmung
- 43: seitliche Ausnehmung
- 44: mittlere Ausnehmung
- 45: schalenförmiges Element
- 46: schalenförmiges Element
- 47: Verbindung
- 48: oberer Rand von 45
- 49: oberer Rand von 46

- 71: Absorptionskörper (Fig. 3A, 3B)
- 79: Rand
- 80: Kernmaterial
- 81: Schicht (undurchlässig)
- 82: Schicht (durchlässig)

- 91: Absorptionskörper (nach dem Stand der Technik Fig. 5)

## Patentansprüche

1. Interlabialer Hygieneartikel enthaltend einen Absorptionskörper (41), zwei längliche flache schalenförmige Elemente (45; 46), deren Längsachsen in X-Richtung und deren Querachsen in Y-Richtung ausgerichtet sind, wobei die Unterseite des größeren schalenartigen Elements die Unterseite des Absorptionskörpers zumindest teilweise bildet,
wobei der Rand (48) des größeren schalenförmigen Elements (45) den Rand des Hygieneartikels bildet,
wobei das kleinere schalenförmige Element (46) im Inneren des größeren schalenförmigen Elements (45) derart angeordnet ist, dass deren Längsachsen im Wesentlichen miteinander fluchten;
wobei die Unterseite des kleineren schalenförmigen Elements (46) an der Oberseite des größeren schalenförmigen Elements an der Verbindungsstelle (47) derart befestigt ist,
dass zwischen der Außenwandung des kleineren schalenförmigen Elements (46) und der Innenwandung des größeren schalenförmigen Elements (45) eine ringförmige Ausnehmung (42, 43) entsteht, wobei in deren zumindest im Mittelbereich des Absorptionskörpers belegene Abschnitte (42, 43), die im Wesentlichen parallel zur Längsachse X verlaufen, die Labiae Maiorae einbringbar sind.

2. Interlabialer Hygieneartikel nach Anspruch 1, **dadurch gekennzeichnet,**
**dass** die Querdimension des inneren schalenförmigen Elements (46) derart gewählt ist, dass der Rand (49) des inneren schalenförmigen Elements zwischen Labia Maiora und Labia Minora anordbar ist.

3. Interlabialer Hygieneartikel nach Anspruch 1 oder 2, **dadurch gekennzeichnet, dass** die Länge des kleineren schalenförmigen Elements (46) 30% bis 70%, vorzugsweise 40% bis 60%, noch bevorzugter etwa die Hälfte der Länge des größeren schalenförmigen Elements (45) in X-Richtung gemessen beträgt und/oder, dass die Breite des kleineren schalenförmigen Elements (46) 30% bis 70%, vorzugsweise 40% bis 60%, noch bevorzugter etwa die Hälfte der Breite des größeren schalenförmigen Elements (45) in Y-Richtung beträgt.

4. Interlabialer Hygieneartikel nach einem der vorstehenden Ansprüche, **dadurch gekennzeichnet, dass** das kleinere, innen befindliche schalenförmige Element (46) in Z-Richtung zumindest gleich hoch ist wie das größere schalenförmige Element (45).

5. Interlabialer Hygieneartikel nach Anspruch 4, **dadurch gekennzeichnet, dass** das kleinere schalenförmige Element (46) höher ist als das größere schalenförmige Element (45), vorzugsweise 10% bis 50%.

6. Interlabialer Hygieneartikel nach einem der vorstehenden Ansprüche, **dadurch gekennzeichnet, dass** die Wandung des inneren schalenförmigen Elements (46) und die Wandung des größeren schalenförmigen Elements (45) aus absorbierendem Material bestehen.

7. Interlabialer Hygieneartikel nach Anspruch 6, **dadurch gekennzeichnet, dass** zumindest die Unterseite des größeren schalenförmigen Elements (45) mit einer flüssigkeitsundurchlässigen Schicht versehen ist.

8. Interlabialer Hygieneartikel nach Anspruch 7, **dadurch gekennzeichnet, dass** die Unterseiten beider schalenförmiger Elemente (45, 46) mit einer flüssigkeitsundurchlässigen Schicht versehen sind.

9. Interlabialer Hygieneartikel nach einem der vorstehenden Ansprüche, **dadurch gekennzeichnet, dass** das kleinere und/oder das größere schalenförmige Element (45, 46) eine flüssigkeitsdurchlässige Obersicht, einen absorbierenden Kern und eine flüssigkeitsundurchlässige Untersicht enthalten.

10. Interlabialer Hygieneartikel nach einem der vorstehenden Ansprüche, **dadurch gekennzeichnet, dass** die Wandung des kleineren schalenförmigen Elements (46) zumindest in ihrem Bodenbereich dicker ist als der Rest von dessen Wandung, um dort eine verstärkte Flüssigkeitsaufnahme zu erreichen.

11. Interlabialer Hygieneartikel nach einem der vorstehenden Ansprüche, **dadurch gekennzeichnet, dass** der Absorptionskörper aus einem blatt- oder bogenförmigen absorbierenden Bahnmaterial gefaltet, geprägt, tiefgezogen oder unter Wärme und Druck geformt ist.

12. Interlabialer Hygieneartikel nach Anspruch 11, **dadurch gekennzeichnet, dass** das Bahnmaterial eine Schicht eines absorbierenden Materials enthält und fakultativ eine flüssigkeitsundurchlässige rückwärtige Schicht und/oder eine flüssigkeitsdurchlässige obere Deckschicht.

13. Interlabialer Hygieneartikel nach Anspruch 11 oder 12, **dadurch gekennzeichnet, dass** das bogenförmige Bahnmaterial biegsam und gleichzeitig eigensteif ist.

14. Interlabialer Hygieneartikel nach einem der vorstehenden Ansprüche, **dadurch gekennzeichnet, dass** die Oberseite des Absorptionskörpers mit einer an sich bekannten, in Längsrichtung verlaufenden Plissierung versehen ist.

15. Interlabialer Hygieneartikel nach einem der vorstehenden Ansprüche, **dadurch gekennzeichnet, dass** der äußere Rand des Absorptionskörpers nach innen eingebogen ist.

16. Interlabialer Hygieneartikel nach einem der Ansprüche 1 bis 14, **dadurch gekennzeichnet, dass** der äußere Rand des Absorptionskörpers nach außen eingebogen ist.

17. Interlabialer Hygieneartikel nach einem der vorstehenden Ansprüche, **dadurch gekennzeichnet, dass** der Absorptionskörper aus einer Schicht eines absorbierenden Materials besteht, die zwischen einer flüssigkeitsdurchlässigen Schicht und einer flüssigkeitsundurchlässigen Schicht angeordnet ist, wobei im Randbereich die flüssigkeitsundurchlässige Schicht um das freie Ende der Schicht aus absorbierendem Material und der Schicht aus flüssigkeitsdurchlässigem Material herumgelegt und an letzterem befestigt ist.

18. Interlabialer Hygieneartikel nach einem der vorstehenden Ansprüche, **dadurch gekennzeichnet, dass** die dem Körper zugewandte (proximale) Seite des Absorptionskörpers eine Beschichtung enthält, die einem Austrocknen der Schleimhäute entgegenwirkt.

## Claims

1. An interlabial hygiene article, comprising an absorption body (41), two elongated flat dish-shaped elements (45; 46) whose longitudinal axes are aligned in X-direction, and whose transverse axes are aligned in Y-direction, with the lower surface of the larger dish-shaped element at least partly forming the lower surface of the absorption body;
with the rim (48) of the larger dish-shaped element (45) forming the rim of the hygiene article;
with the smaller dish-shaped element (46) in the interior of the larger dish-shaped element (45) being arranged such that their longitudinal axes are essentially aligned in relation to each other;
with the lower surface of the smaller dish-shaped element (46) being attached to the upper surface of the larger dish-shaped element at the junction (47);
such that a ring-shaped recess (42, 43) is created between the outer wall of the smaller dish-shaped element (46) and the inner wall of the larger dish-shaped element (45); with the labiae majorae being accommodatable in the sections (42, 43) of the ring-shaped recess, which sections are situated at least in the central region of the absorption body and which sections extend essentially parallel to the longitudinal axis X.

2. The interlabial hygiene article according to claim 1, **characterized in that**
the transverse dimension of the inner dish-shaped element (46) is such that the rim (49) of the inner dish-shaped element can be arranged between the labia majora and labia minora.

3. An interlabial hygiene article according to claim 1 or 2, **characterized in that** the length of the smaller dish-shaped element (46) is 30 % to 70 %, preferably 40 % to 60 %, more preferably approximately half the length of the larger dish-shaped element (45), measured in the X-direction; and/or that the width of the smaller dish-shaped element (46) is 30 % to 70 %, preferably 40 % to 60 %, more preferably approximately half the width of the larger dish-shaped element (45), measured in the Y-direction.

4. The interlabial hygiene article according to one of the preceding claims, **characterized in that** the smaller inner dish-shaped element (46) is at least of the same height as the larger dish-shaped element (45) in the Z-direction.

5. The interlabial hygiene article according to claim 4, **characterized in that** the smaller dish-shaped element (46) is higher than the larger dish-shaped element (45), preferably 10 % to 50 % higher.

6. The interlabial hygiene article according to one of the preceding claims, **characterized in that** the wall of the inner dish-shaped element (46) and the wall of the larger dish-shaped element (45) are made of absorbent material.

7. The interlabial hygiene article according to claim 6, **characterized in that** at least the lower surface of the larger dish-shaped element (45) comprises a fluid-impermeable layer.

8. The interlabial hygiene article according to claim 7, **characterized in that** the lower surfaces of both dish-shaped elements (45, 46) comprise a fluid-impermeable layer.

9. The interlabial hygiene article according to one of the preceding claims, **characterized in that** the smaller and/or the larger dish-shaped element (45, 46) comprise a fluid-permeable upper layer, an absorbent core, and a fluid-impermeable lower layer.

10. The interlabial hygiene article according to one of the preceding claims, **characterized in that** the wall of the smaller dish-shaped element (46) at least in its bottom region is thicker than the remaining wall, so as to achieve improved absorption of fluids in said region.

11. The interlabial hygiene article according to one of the preceding claims, **characterized in that** the absorption body is made of a web of a sheet-like absorbent material which is folded, embossed, deep-drawn or deformed by heat and/or pressure.

12. The interlabial hygiene article according to claim 11, **characterized in that** the sheet material comprises a layer of an absorbent material and, optionally, a fluid-impermeable backing layer and/or a fluid-permeable upper cover layer.

13. The interlabial hygiene article according to claim 11 or 12, **characterized in that** the web of sheet-like material is flexible and at the same time inherently rigid.

14. The interlabial hygiene article according to one of the preceding claims, **characterized in that** the upper surface of the absorption body is pleated in longitudinal direction, in a way which is known per se.

15. The interlabial hygiene article according to one of the preceding claims, **characterized in that** the outer rim of the absorption body is bent over inwards.

16. The interlabial hygiene article according to one of claims 1 to 14, **characterized in that** the outer rim of the absorption body is bent over outwards.

17. The interlabial hygiene article according to one of the preceding claims, **characterized in that** the absorption body comprises a layer of an absorbent material, said layer being arranged between a fluid-permeable layer and a fluid-impermeable layer, with the fluid-impermeable layer in the region of the rim being placed around the free end of the layer made of absorbent material and the layer made of fluid-permeable material, and being attached to the latter material.

18. The interlabial hygiene article according to one of the preceding claims, **characterized in that** the surface of the absorption body facing the wearer's body, i.e. the proximal surface of the absorption body, is treated so as to prevent any drying out of the mucous membranes.

## Revendications

1. Article d'hygiène interlabial contenant un corps d'absorption (41), deux éléments allongés plats en forme de coupelle (45, 46) dont les axes longitudinaux sont orientés dans la direction X et dont les axes transversaux sont orientés dans la direction Y, où la face inférieure du plus grand élément en forme de coupelle forme la face inférieure du corps d'absorption au moins partiellement,
dans lequel le bord (48) du plus grand élément en forme de coupelle (45) forme le bord de l'article d'hygiène,
dans lequel le plus petit élément en forme de coupelle (46) est disposé à l'intérieur du plus grand élément en forme de coupelle (45) de façon telle que leurs axes longitudinaux sont essentiellement alignés l'un avec l'autre ;
dans lequel la face inférieure du plus petit élément en forme de coupelle (46) est fixée sur la face supérieure du plus grand élément en forme de coupelle au niveau du point de liaison (47),
**caractérisé en ce qu'**entre la paroi externe du plus petit élément en forme de coupelle (46) et la paroi interne du plus grand élément en forme de coupelle (45) se forme un évidement annulaire (42, 43), suite à quoi les grandes lèvres peuvent être insérées dans ses segments (42, 43), placés au moins dans la zone médiane du corps d'absorption, qui s'étendent essentiellement parallèlement à l'axe longitudinal X.

2. Article d'hygiène interlabial selon la revendication 1, **caractérisé**
**en ce que** la dimension transversale de l'élément interne en forme de coupelle (46) est sélectionnée de façon telle que le bord (49) de l'élément interne en forme de coupelle peut être disposé entre les grandes lèvres et les petites lèvres.

3. Article d'hygiène interlabial selon la revendication 1 ou 2, **caractérisé en ce que** la longueur du plus petit élément en forme de coupelle (46) représente 30 % à 70 %, de préférence 40 à 60 %, de façon encore plus préférée environ la moitié de la longueur du plus grand élément en forme de coupelle (45) telle que mesurée dans la direction X et/ou **en ce que** la largeur du plus petit élément en forme de coupelle (46) représente 30 % à 70 %, de préférence 40 % à 60 %, de façon encore plus préférée environ la moitié de la largeur du plus grand élément en forme de coupelle (45) dans la direction Y.

4. Article d'hygiène interlabial selon l'une des revendications précédentes, **caractérisé en ce que** le plus petit élément en forme de coupelle se trouvant à l'intérieur (46) dans la direction Z est au moins aussi haut que le plus grand élément en forme de coupelle (45).

5. Article d'hygiène interlabial selon la revendication 4, **caractérisé en ce que** le plus petit élément en forme de coupelle (46) est plus haut que le plus grand élément en forme de coupelle (45), de préférence de 10 à 50 %.

6. Article d'hygiène interlabial selon l'une des revendications précédentes, **caractérisé en ce que** la paroi de l'élément interne en forme de coupelle (46) et la paroi du plus grand élément en forme de coupelle (45) se composent de matériau absorbant.

7. Article d'hygiène interlabial selon la revendication 6, **caractérisé en ce qu'**au moins la face inférieure du plus grand élément en forme de coupelle (45) est munie d'une couche imperméable au fluide.

8. Article d'hygiène interlabial selon la revendication 7, **caractérisé en ce que** les faces inférieures des deux éléments en forme de coupelle (45, 46) sont munies d'une couche imperméable au fluide.

9. Article d'hygiène interlabial selon l'une des revendications précédentes, **caractérisé en ce que** le plus petit et/ou le plus grand élément en forme de coupelle (45, 46) contient (contiennent) une couche supérieure perméable au fluide, un noyau absorbant et une couche inférieure imperméable au fluide.

10. Article d'hygiène interlabial selon l'une des revendications précédentes, **caractérisé en ce que** la paroi du plus petit élément en forme de coupelle (46) est plus épaisse au moins dans sa zone de fond que le reste de sa paroi pour y atteindre une captation de fluide renforcée.

11. Article d'hygiène interlabial selon l'une des revendications précédentes, **caractérisé en ce que** le corps d'absorption en un matériau de bande absorbante en forme de feuille ou de courbe est plié, estampé, embouti ou formé sous pression et chaleur.

12. Article d'hygiène interlabial selon la revendication 11, **caractérisé en ce que** le matériau de bande contient une couche d'un matériau absorbant et facultativement une couche arrière imperméable au fluide et/ou une couche de recouvrement supérieure perméable au fluide.

13. Article d'hygiène interlabial selon la revendication 11 ou 12, **caractérisé en ce que** le matériau de bande en forme de courbe est flexible et a simultanément une rigidité propre.

14. Article d'hygiène interlabial selon l'une des revendications précédentes, **caractérisé en ce que** la face supérieure du corps d'absorption est munie d'un pliage connu en soi s'étendant dans la direction longitudinale.

15. Article d'hygiène interlabial selon l'une des revendications précédentes, **caractérisé en ce que** le bord externe du corps d'absorption est replié vers l'intérieur.

16. Article d'hygiène interlabial selon l'une des revendications 1 à 14, **caractérisé en ce que** le bord externe du corps d'absorption est replié vers l'extérieur.

17. Article d'hygiène interlabial selon l'une des revendications précédentes, **caractérisé en ce que** le corps d'absorption se compose d'une couche d'un matériau absorbant qui est disposée entre une couche perméable au fluide et une couche imperméable au fluide, où dans la zone de bord, la couche imperméable au fluide est placée autour de l'extrémité libre de la couche en matériau absorbant et de la couche en matériau perméable au fluide et fixée à cette dernière.

18. Article d'hygiène interlabial selon l'une des revendications précédentes, **caractérisé en ce que** la face orientée vers le corps (proximale) du corps d'absorption contient un revêtement qui contre un assèchement des muqueuses.
